(19) Europäisches Patentamt

European Patent Office

Office européen des brevets

(11) **EP 1 723 904 A1**

(12) **EUROPEAN PATENT APPLICATION**
published in accordance with Art. 158(3) EPC

(43) Date of publication:
**22.11.2006 Bulletin 2006/47**

(51) Int Cl.:
***A61B 5/00*** (2006.01)

(21) Application number: **05714821.5**

(86) International application number:
**PCT/CN2005/000290**

(22) Date of filing: **10.03.2005**

(87) International publication number:
**WO 2005/084528 (15.09.2005 Gazette 2005/37)**

(84) Designated Contracting States:
**AT BE BG CH CY CZ DE DK EE ES FI FR GB GR HU IE IS IT LI LT LU MC NL PL PT RO SE SI SK TR**

(30) Priority: **10.03.2004 CN 200410008403**

(71) Applicant: **Liu, Zhongqi
Beijing 100080 (CN)**

(72) Inventor: **Liu, Zhongqi
Beijing 100080 (CN)**

(54) **A METHOD FOR EVALUATING THE EFFECT OF MODULATORY MEANS FOR PHYSIOLOGIC/ PSYCOLOGIC CONDITIONS**

(57) The present invention relates to a method of effect evaluation of adjustment measures on human body's physiological and psychological states by means of using Thermal Texture Mapping Technology (TTM), the said adjustment measures comprise one or more of taken-in substances, physical stimulations and mental treatments which are applied actively or passively onto human body and could bring about some changes to human body's physiological and psychological states; Before and after applying one or more of the adjustment measures, the infrared thermal radiation intensities of the parts, organs and/or tissues of the human body in concern are determined and from them the differential values between the thermal radiation intensities of the same abnormal parts, organs and/or tissues before and after application of the adjustment measure (s) are obtained, so as to evaluate the effect of the adjustment measures applied onto physiological and psychological states of the human body. The inventive method has the advantages of being safe, real-time, dynamic, quick, accurate, economical and convenient, and has a very good application prospect.

EP 1 723 904 A1

**Description**

Technical Field

This invention relates to an evaluative method of effect of adjustment measures on human body's physiological and psychological states, in particular to an evaluative method of effect of adjustment measures on human body's physiological and psychological states by means of using Thermal Texture Maps Technology (TTM).

Background Technology

It is a common topic for human beings to pay attention to their own healthy state, mainly physiological and psychological healthy states. A large number of exchanges and transfers of substance, energy and information take place between human body and the nature as well as the society, so human beings' physiological and psychological states have much to do with inheritance, natural environment and living conditions such as diet, work, and applied physiological and psychological adjustment measures. People's physiological and psychological states could now be roughly divided into healthy state, sub-healthy state, and abnormal state, each of which can be further divided into many different stages.

Strictly speaking, no one is in an absolute physiological and psychological healthy state, because there are many physiological levels of human body such as gene, molecule, cell, organs, tissue, parts of body and the human body as a whole, and they have to undergo different physiological and psychological growth stages, therefore, different effects and reflection may occur when human body is applied with outside adjustment measures. All these will lead to the human body's deviation from physiologically and psychologically healthy states in some period at certain level.

Therefore, people always hope to understand changing trend and rules and to control their physiological and psychological states. Some adjustment measures through taking-in substances, exerting physical stimulations and mental treatments have been developed to adjust human body's physiological and psychological states, in a hope of improving and strengthening the physiological physique and psychological quality.

In order to comprehend the influence of adjustment measures, which are actively applied to or passively accepted by human body, on physiological and psychological states and its degree, it will at least involve the evaluation of the changes of human body's physiological and psychological states after having received one or more of the following three kinds of adjustment measures.

The first kind of adjustment measures mainly refers to certain taken-in substances which could cause some changes of human body's physiological and psychological states. The so called certain taken-in substances comprises orally administrated and externally applied substances, including but not limited to one or more of the following types of substances which are actively administrated or applied: medicine, tonics, nutriment, body care products such as cosmetics, beauty making-up, etc,, enhancer agent, food therapy articles; or one or more of the following types of substances which are actively or passively taken-in or applied: cigarette smoking, alcohol, harmful substances which cause dependence or obviously disturbs to human body's normal physiological and psychological functions; pollutants and/or toxic substances contained in atmosphere, drinking water, and/or food; misuse, overuse of the medicine, tonics, nutriment, body care products, enhancer agent, food therapy articles; the said administration, application or acceptance of the said substances comprises oral administration, injection, percutaneous adsorption or taking-in by smoking or respiration, etc.

The second kind of adjustment measures mainly refers to those physical stimulations which have been actively or passively accepted by and could cause physiological and psychological changes to human body. The said physical stimulations include but is not limited to one or more of the following actively accepted physical stimulations: keep-fit massage, health-aimed sports, acupuncture-moxibustion, digit-pressing moxibustion, electromagnetic spectrum therapy, needle embedding, meditation and qigong excises; or one or more of the passively accepted physical stimulations: electromagnetic field, light- and radioactive pollution; (ultra)sound wave and noise pollution; harms and disability caused by outside force; bad living and working conditions, habits and ways.

The third kind of adjustment measures mainly refers to those actively or passively accepted mental treatments which could bring about physiological and psychological changes to human body. The said mental treatments include but not limited to one or more of the following actively exerted ones: moral encouragement, consolation, physiological therapy, hypnotism, qigong exercise, religious reformatory; or one or more of the following passively accepted ones: fright, threatening and irrational mental control, as well as the unhealthy environment or relationship with others which could lead to a mood of constrain, sorrow, decline, despair, hatred; IQ test and lie detection, etc.

The cases in which human body's physiological and psychological states need to be evaluated are far more than the above-mentioned; but as for the above- listed, huge demands have been existed.

Known from the prior art technologies, some measures and methods have been developed to evaluate human body's physiological and psychological states for different practical purposes, but all these prior art technologies have a common defect, that is, it cannot demonstrate characteristic co-relation, in a direct, real-time, dynamic and in situ way and at the level of cell function, between the human body's physiological/psychological changes and the exerted adjustment measures such as substances taken-in, physical stimulations and/or mental treatments.

In contrast, the prior art evaluative methods often need to be manifested indirectly and/or after a comparatively long period of time when some adjustment measures have been applied onto human body, and/or through human body's expression of his/her subjective feelings, and/or through some physiological/physiochemical quota; and what's more, they cannot demonstrate characteristic co-relation between the said adjustment measures and the human body's physiological/ psychological states , in many cases.

For instance, before a new chemical medicine is promoted to the market, it will have taken from several to more than ten years to evaluate its pharmacodynamical effectiveness, short term and long term toxic and side effects, possibility of causing deformity to human body, etc., and a lot of labor and material resources will have been spent, but it still cannot guarantee a safe use after it is marketed. Some side effects of new medicine could only be found after it has been used for a long period of time, which might some time lead to cancellation of its market license by FDA.

For another example, for stimulant control, it mainly depended on analysis on the athlete's urinate- or blood sample, in the past, to determine whether an athlete had taken-in stimulant before a contest or not. Such kind of method has the problem of indirectness, time wasting and high cost, and the course of sampling is not so convenient and is non-real time.

Since the infrared thermal tissue mapping technology (referred to hereafter as TTM) and its implementing equipment have been provided by Professor Mr. Liu, Zhong Qi in US Patent No. 6,023,637, human beings have obtained for the first time an effective tool for functional evaluation on human body's physiological and psychological states at the level of cell functional vitality. By means of using TTM technology, it is possible to overcome the defects of the prior art methods, and to solve the problems existed before, and TTM represents a break-through of functional evaluation onto human body's physiological and psychological states.

Description of Invention

One object of the invention is to provide an evaluative method of effect of adjustment measures on human body's physiological and psychological states after having applied adjustment measures, including taking-in substances, applying physical stimulations and/or mental treatments onto human body by means of using the TTM technology.

Another object of this invention is to provide a use of TTM technology for evaluation of adjustment measures on the human body's physiological and psychological states.

The relevant contents of the TTM technology and equipments used in this invention could be seen from the US Patent No. 6,023,637, Chinese Patent No. ZL02235271, ZL02234963, ZL02308058, and Chinese patent application No. 03276575.4 and No. 03276576.2. The said relevant contents are hereby incorporated into the present application by reference.

The terms "taking-in/taken-in, exerting/exerted and applying/application" used in this application each means to let the human body accept an action, including actively or passively accepted action by way of either touching onto the body surface, taking-in certain substances by oral administration, injection, percutaneous adsorption; by smoking and/or respiration, etc; the term "adjustment measures " means those measures which could cause either positive or negative changes to human body's physiological and psychological states; The term "human body's physiological and psychological states" means the physiological and psychological states of the human body at the level of either the body as a whole, its parts, organs and/or tissues, the said states are mainly indicated by the relative strength of the cell metabolism level represented by the level of cell's infrared thermal radiation.

Not hope to be bound by any theory, the inventor believes that, according to the systematology, human body is a big system, which continually keeps exchanges of substance, energy and information with other bigger systems like nature and society to guarantee and renew the operation of the human body system. A change of any part of human body system is sure to have impact on the human body as a whole, and an observation of the changes of the states of the human body as a whole can most help correct understandings, in an all round way, in positive and side effect brought about by applying adjustment measures, this point of view is so called "entirety theory". Each subsystem of human body relates closely to and coordinates mutually with the others, and there are exchanges of substance, energy and information among these subsystems, the states of a subsystem could also be indicated and reflected by certain other subsystems, this is called "holography theory" of the human body. To regard the central line from the top of the head to the perineum as the centre axis, a healthy human body shall have a relatively balanced intensity of thermal radiation on the left and right sides, and should not appear a state of obvious imbalance, this is called the "symmetry theory" of the human body. Under a healthy state, there is an automatic mechanism of mutual promotion and restriction among each subsystem of human body, in order to keep the mutual exchanges of substance, energy and information basically stable, and to keep metabolism of each organ in a balanced and stable state, this is called the "balance theory" of the human body. Human body remains a vital state, the cells are keeping in continuous metabolism, the microcosmic structure and functions of organs, tissues and human body as a whole, which are made up of alive cells, remain changing and keep renewing, so it is obvious that useful or harmful changes always occur under the influences of the factors of inheritance, age and adjustment measures, etc; This also indicates that it is possible for human beings to actively apply some adjustment measures so as to positively adjust his/her physiological and psychological states, this is called the "dynamic theory" of human body's states.

According to the entirety theory and dynamic theory, the inventor holds the view that, the human body, as a big system, always keeps exchanges of substance, energy and information with the outside world, and each of its subsystem also keeps exchanges of substance, energy and information with the others, this is the premise to maintain a vital human body having physiological and psychological functions. So some actions exerted from the outside world may obviously, actively or negatively change the physiological and psychological states of the human body as a whole and/or of its parts. In contrast, we can tell more precisely the effect of the adjustment measures onto the physiological and psychological states of the human body through observation on functions of each subsystem, especially of the changes of the physiological and psychological states of the human body as a whole, so that we can understand correctly and make active use of positive effects, and avoid negative or side effect to the physiological and psychological states, which could be brought about to the human body by the said adjustment measures.

The inventor chooses people's lips, navel and internal secretion system as the three representative windows for entire vitality/abnormal states of the human body as a whole, wherein the lips' thermal radiation is regarded as an indication of human body's nutrition-work-rest balance state, and is also regarded as the main evaluative parameter of recovering state from diseases; and wherein the thermal radiation of the navel is regarded as an important indicative window for vitality of the human body as a whole and of the healthy state of the lower part of the human body; and wherein the cells' metabolism state of the internal secretion system including the endocrine glands such as hypophysis cerebri, thyroid gland, prostate/ovary, pancreas, microcirculation, etc, is the important indicative window of crucial imbalance of the human body. The inventive method thus firstly detects and analyzes the degree of thermal radiation as well as its changing trend of these three representative windows, in order to understand the human body's physiological and psychological function states from a comprehensive standpoint.

According to the balance theory, the inventor believes that among the subsystems, i.e., parts, organs, and tissues of the human body, there is always a mutually promoting and restrictive stabilizing balance mechanism through exchanges of substance, energy and information, while the human body's endocrine glands play a prevailing role in the stabilizing balance mechanism.

The mentioned endocrine glands include thalamencephalon, thyroid gland, mammary gland, pancreas, prostate/adrenal gland, ovary, etc, as well as the secreted substances thereof. They determine the kind and level of the exchanges of substance, energy and information among the subsystems; a change of function of the internal secretion system is sure to lead to certain change of the function of the related subsystems, organs and/or tissues, so the thermal radiation level of the internal secretion system is an indicative window to observe whether the function of the subsystems is in a stable balance state or not, and whether there is a trend of occurring serious diseases or not. The balance theory is also applicable to analyze the functions of each organ, tissue, that is to say, under normal conditions and in the same organ or tissue, each part should remain in a thermally balanced and stable state. If the metabolism of some cells in certain part of human body becomes abnormal, its thermal radiation level is sure to be obviously different from that of the neighbor parts, and this certainly indicates an abnormal function of the part. Therefore, it establishes a corresponding co-relation between the abnormal function and abnormal thermal radiation.

Nine regional indicative windows are selected for standing for human body's regional physiological and psychological functions , including six zones on the front part of human body forming three pairs in a left-right symmetric relation, i.e., the two clavicle pits, the two axillae and the two inguina ditch, as well as the upper, middle and lower sector s on the back spinal line of human body. The five differential values of the thermal radiation level of the three symmetric pairs and of the thermal radiation level of the said upper-middle sector pair and middle-lower sector pair on the back spinal line are therefore taken to respectively represent their lumped performances of the functions of the main organs, tissues of human body, in an entirety point of view. Each of the nine regional indicative windows could indicate respectively and correspondingly the physiological functions of the following parts, organs, tissues of human body:

Left clavicle pit: left part of head, left part of neck, left lung;
Right clavicle pit: right part of head, right part of neck, right lung;
Left axilla: left breast, duodenum, esophagus, stomach, pancreas, left kidney, spleen;
Right axilla: right breast, liver, gallbladder, right kidney;
Left inguina ditch:

Upper sector: descending colon, rectum;
Middle sector: pelvic cavity, adnexitis, bladder;
Lower sector: prostate, uterus, vagina;

Right inguina ditch:

Upper sector: ascending colon, cecum , pelvic cavity, adnexitis;
Middle sector: pelvic cavity, adnexitis, bladder;
Lower sector: uterus, prostate, adnexitis.

Spinal: it can not only indicate the diseases of neck, chest, waist, sacrum, but can also indicate correspondingly the physiological states of the following parts, organs, tissues:

Upper sector: lung, gallbladder;
Middle sector: kidney, stomach, lung;
Lower sector: rectum, uterus, prostate, anus.

When any one of the five thermal differential values taken from the nine regional windows is larger than 0.2, then it indicates that at least one of the parts, organs, tissues represented by the zone having the higher thermal radiation parameter performs an abnormal physiological function, and it is necessary to analyze these parts, organs, tissues one by one with help of TTM technology, and to find out the abnormal part, organ or tissue, and to supervise changing trend of the abnormal thermal radiation when certain adjustment measure is applied.

The following steps are comprised according to the inventive method:

(1) Before certain adjustment measures are applied, conducting a scanning on the human body surface for a record of a thermal radiation mapping, taking a maximum value of the thermal radiation of each part, organs and tissues as its representative $F_i^0$, i = 1 , 2 , ... , I, in an unit of °C;I is the total number of the parts, organs or tissues, from which corresponding thermal radiation numerical values are measured by means of using TTM technology; thereby obtaining the thermal radiation intensity of the respective parts $F_i^0$, i=1 , 2 , ... , I;

(2) Taking the thermal radiation numerical values of the lips, navel, and each endocrine glands, which are marked respectively as $F_{lips}^0$, $F_{navel}^0$, $F_{gland\,l}^0$, 1= 1 , ... L, L is the number of the endocrine glands to be tested; these $F_{lips}^0$, $F_{navel}^0$, $F_{gland\,l}^0$, 1=1 , ...L are used to evaluate human body's original physiological states of human body as a whole before application of any adjustment measure; a healthy human body should has $F_{lips}^0$ =0±0.2, $F_{navel}^0$ =2.0±0.2, $F_{gland\,l}^0$ ≤0.1;

(3) Obtaining the nine thermal radiation values, marking them as $F_r^0$ , r = 1 , 2 , ... , 9, from the nine regional indicative windows, that is, two clavicle pit, two axillae and two inguina ditch on the left and right side of the body's centre axis respectively, and from the upper, middle and lower sector of the back spinal; Each parameter respectively stands for as the following:

$F_1^0$ :    left clavicle pit;           $F_2^0$ :    right clavicle pit;

$F_3^0$ :    left axilla;           $F_4^0$ :    right axilla;

$F_5^0$ :    left inguina ditch;           $F_6^0$ :    right nguina ditch;

$F_7^0$ :    upper sector of the spinal           $F_8^0$ :    middle sector of the spinal

$F_9^0$ :    lower sector of the spinal;

and figuring out following 5 absolute differential values of the five symmetric pairs from the corresponding nine thermal radiation numerical values:

$$\Delta F_1^0 = \left| F_1^0 - F_2^0 \right|$$

$$\Delta F_2^0 = \left| F_3^0 - F_4^0 \right|$$

$$\Delta F_3^0 = \left| F_5^0 - F_6^0 \right|$$

$$\Delta F_4^0 = \left| F_7^0 - F_8^0 \right|$$

$$\Delta F_5^0 = \left| F_8^0 - F_9^0 \right|$$

if any one among $\Delta F_k^0$, k = 1 , ... , 5 is larger than or equal to 0.2, it then needs to further analyze and obtain, with help of TTM technology, the thermal radiation intensity of each and all parts, organs and tissues represented by the zones having a higher thermal radiation numerical value, so as to find out the parts having abnormal function, and to figure out the thermal radiation numerical values $F_{ot}^0$, t = 1 , 2 , ... , T ; T is the number of the abnormal parts, organs and tissues;

(4) Letting a human body accept one or more adjustment measures;

(5) In J time intervals, conducting scanning on human body surface for J records of the thermal radiation mapping with help of TTM technology, thereby obtaining J batches of the thermal radiation intensity of the respective parts.

$$F_{lips}^j \text{、} \quad F_{navel}^j \text{、} \quad F_{\text{gland } l}^j \text{ , } l = 1 \text{ , } \dots \text{ , } L \text{ ;}$$

$$\Delta F_1^j \text{、} \quad \Delta F_2^j \text{、} \quad \Delta F_3^j \text{、} \quad \Delta F_4^j \text{、} \quad \Delta F_5^j \text{ ; And}$$

$$F_i^j \text{ , i=1 , 2 , } \dots \text{ , I ; j = 1 , 2 , } \dots \text{ , J;}$$

(6) Figuring out the followings:

$$\Delta F_{lips}^j = F_{lips}^j - F_{lips}^0 \text{ ;}$$

$$\Delta F_{navel}^j = F_{navel}^j - F_{navel}^0 \text{ ;}$$

$$\Delta F_{\mathrm{gland\,l}}^{j} = F_{\mathrm{gland\,l}}^{j} - F_{\mathrm{gland\,l}}^{0} \quad , \mathrm{l} = 1 \; , \; 2 \; , \; \ldots \; , \; \mathrm{L;}$$

$$\Delta(\Delta F_{k})^{j} = \Delta F_{k}^{j} - \Delta F_{k}^{0} \quad , \mathrm{k} = 1 \; , \; 2 \; , \; 3 \; , \; 4 \; , \; 5 \; ;$$

And

$$\Delta F_{\mathrm{ot}}^{j} = F_{\mathrm{ot}}^{j} - F_{\mathrm{ot}}^{0} \quad , \mathrm{t} = 1 \; , \; 2 \; , \; \ldots \; , \; \mathrm{T} \; ;$$

$$\mathrm{j} = 1 \; , \; 2 \; , \; \ldots \; , \; \mathrm{J} \; ;$$

(7) As for the newly found Q parts having abnormal function clued by obviously increased $\Delta(\Delta F_k)^j$, k = 1, 2, 3, 4, 5 ; j = 1, 2, ... J in step
(6) conducting TTM analysis and determining the followings:

$$\Delta F_{\mathrm{q}}^{j} = F_{\mathrm{q}}^{j} - F_{\mathrm{q}}^{0} \quad , \mathrm{q} = 1 \; , \; 2 \; , \; \ldots \; , \; \mathrm{Q} \; ; \mathrm{j} = 1 \; , \; 2 \; , \; \ldots \; , \; \mathrm{J} \; ;$$

(8) Using the differential values of thermal radiation intensity $\Delta F^j$ and $\Delta(\Delta F)^j$, j = 1, ...J, obtained in steps (6) and (7), for the purpose of determining the positive and side effect of the adjustment measures applied onto the human body as a whole, onto regional zones and individual organs, tissues, and determining the starting time point and the lasting period of the effectuation according to the criteria below:.

    $0\leq$ and $\leq0.1$, having side effect, but not obvious
    $>0.1$ and $\leq0.2$, obvious side effect
    $>0.2$ and $\leq0.3$, strong side effect
    $>0.3$ and $\leq0.4$, very strong side effect
    $\geq0.5$ poison;
    $\leq0$ and $\geq-0.1$, positive effect, not clear
    $<-0.1$ and $\geq-0.2$, obvious positive effect
    $<-0.2$ and $\geq-0.3$, strong positive effect
    $<-0.3$ and $\geq-0.4$, very strong positive effect
    $< -0.5$ best effect.

These parameters indicate not only the positive and side effect rendered by applied adjustment measures onto human body at different levels, but also reflect the starting time point and lasting time of the effectuation.

In addition, the inventive method could be used to evaluate adjusting effect to the physiological states of human body through determining the thermal radiation intensity of the corresponding acupuncture points in the collateral channels system of human body after being applied with certain adjustment measures, and thereby to evaluate the changes of the physiological functions of the parts, organs and tissues which have inherent and corresponding physiological relation with the acupuncture points.

Some adjustment measures can manifest obviously positive adjustment effect or even better at the three levels, i.e., on human body as a whole, regional zones, organs or tissues at the same time, even meanwhile have obviously positive effect or better on several organs and/or tissues, or have specially good effect on certain organ or tissue, while have no obvious side effect on other organs, tissues. This is the best situation. Some adjustment measures have obviously positive effect on a certain organ and/or tissue, but meanwhile have observable but not so strong side effect on other organ or tissue; These adjustment measures can be applied under restricted conditions; in case side effect of some adjustment measures is superior to positive effect, these measures might be taken only if it is in an urgent case or there isn't other better choice, and some other measures need to be used to moderate or offset the side effect; but some adjustment measures have quite strong side effect and the positive effect is not strong, such adjustment measure should be discarded in principle.

According to the changes of determined $\Delta F^j$, j=1 , 2 , ... , J in different time intervals, it can get to know the lasting time of the adjusting effect to the physiological states by some adjustment measures at the three levels of human body as a whole, regional zones and organs/tissues of human body, thus comparatively accurate and objective time intervals for applying some adjustment measures , e.g., the times of taking-in medicine or doing the health exercises per day, to maintain sufficient adjustment effect can be determined. People can also get to know the acceptable maximum and best quantity/intensity of some adjustment measures from the changes of determined $\Delta F^j$, j=1 , 2 , ... , J in different time intervals, wherein the quantity/intensity of applied adjustment measures which are capable of bringing about an obvious positive effect can be regarded as the minimum effective quantity/intensity, and, on the basis of this minimum effective quantity/intensity, an increment of the applied quantity/intensity of the adjustment measures is given gradually, so as to observe whether or not a corresponding increase of positive effect occurs at the same pace, and this process is proceeded till to an extent at which an increase of the positive effect could no longer be observed, the corresponding quantity/intensity of the applied adjustment measures to the said extent can be regarded as the maximum quantity/ intensity applicable to the human body. Having considered comprehensively the factors of the physique, instant physiological and psychological states of, and side effect of the adjustment measures to the human body to be adjusted, an initial optimized quantity/intensity of the adjustment measures to be applied can be ascertained, and, on this basis, an optimized supplementary quantity/intensity at suitable intervals can be applied so as to maintain a best effect. Of course, same principle and procedure can be applied to ascertain an initial and supplementary optimized quantity/intensity of some other adjustment measures which are aimed to offset the side effect of the main adjustment measures.

Similarly, when evaluating the negative effects of those adjustment measures onto physiological and psychological states of human body which people passively accepts, like atmosphere-, water- and food pollution, some harmful working environment in some areas, bad working and living ways and habits, harmful influence of electromagnetic field and radioactive radiation, (ultra)sound wave, etc, people can evaluate the degree of the side effect of the said adjustment measures from the parameters of $\Delta F^j$, j = 1 , 2 , ... , n , and then set up a set of quality- and/or quantity- standard for safety control, so as to prompt and alarm people to avoid actively or to control possible side effect within a safe level.

As for different adjustment measures applied, the time intervals for TTM analysis could be different. Generally speaking, the first time and time intervals for evaluating medicine and other diseases-preventive products, which could have quick and comparative strong adjustment effects, should be comparatively short. For example, the shortest first time for an evaluation could be 20 minutes after application of the adjustment measures, and then followed up with evaluations at certain time intervals, for instance, at the time points of 40th minute, one hour, four hours, eight hours, twelve hours, twenty-four hours. The length of individual time interval should be adjusted dynamically according to the changing trend of the thermal radiation values obtained at the preceding time points. Usually, at those time points, at which obvious changes of the thermal radiation values could be observed and at which the thermal radiation values reaches a stable level, evaluations are better to be " conducted, and these time points are regarded as representative time points of thermal radiation-time profile.

As for some adjustment measures inclusive of health care products such as tonics, nutriment, body care products, enhancer agent, food therapy product and for some physical stimulations, which have comparatively slow adjustment effects on human body and need to be applied in a longer period of time, the first evaluation should be conducted no later than one hour after application of the adjustment measure, and then, based on the difference between the first thermal radiation value obtained after applying the adjustment measure and that obtained before applying the adjustment measure, determination of the time intervals for followed up evaluation of the thermal radiation values after first application of the adjustment measure, and of the time intervals for followed up applications of the adjustment measure. Generally speaking, the effect of this adjustment measure is relatively slow and gentle, so the time intervals can be comparatively long, and the same or similar principles and procedures mentioned above for carrying out the inventive method are also applicable in this circumstance.

As for the evaluation on effect of some mental treatment measures onto physiological and psychological states of human body, the result may vary significantly depending on the physique and diathesis of the individuals. The principle and procedures for determining time intervals are the same as that mentioned above.

Having got to know the contents of the present invention disclosed above, a skilled in the art could easily understand that the inventive method has the advantages of being safe, real-time, dynamic, quick, accurate, economical and convenient, and has a very good application prospect.

In the following, the present invention is further described with the help of some embodiments. However, these embodiments shall not be regarded as any restriction to the scope of the invention. Any modification or variation, which may be made by skilled in the art based on the disclosure of the present application without a creative effort and which does not deviate from the spirits of the inventive concept, falls within the scope of the present invention as defined in the attached claims.

Embodiments

Example 1

According to one embodiment of this invention, a TTM apparatus of model TSI-21M, which is manufactured and available from Beijing Bioyear Co. Ltd., is used to evaluate the adjustment effect of a food therapy product, branded as "Rou Zhi Mei emulsifiable particulate" made from natural plants for preventing hyperplasia of mammary glands. This product is manufactured and available from Compt Institute of Bio-science.

This product is administrated orally after being mixed with drinking water at a dosage of three times per day, 20g per time.

Taking 30 women at an age of from 18 to 55 years old and having lobular hyperplasia confirmed by clinical diagnosis in a test group; Before and after administration of the product, each person is subject to a TTM scanning onto the whole body surface, and the data from the TTM analysis at the time points of 60 minutes, 72 hours, one week, two weeks after the administration are listed as follows:

| Time Interval | Check item Effect | $\triangle F_{breast}$ (Person) | $\triangle F_{axilla}$ (Person) | $\triangle F_{lips}$ (Person) | $\triangle F_{navel}$ (Person) | $\triangle F_{gland}$ (Person) | $\triangle F_{liver}$ (Person) | $\triangle F_{kidney}$ (Person) |
|---|---|---|---|---|---|---|---|---|
| 60 minutes | $\leqslant -0.5$ | 1 | 0 | 0 | 0 | 0 | 0 | 0 |
| | $\leqslant -0.4$ | 2 | 2 | 0 | 0 | 1 | 0 | 0 |
| | $\leqslant -0.3$ | 10 | 8 | 0 | 0 | 2 | 0 | 0 |
| | $\leqslant -0.2$ | 17 | 19 | 0 | 0 | 27 | 0 | 0 |
| | $\pm 0.1$ | 0 | 0 | 30 | 30 | 0 | 30 | 30 |
| | $\geqslant 0.2$ | 0 | 0 | 0 | 0 | 0 | 0 | 0 |
| 72 hours | $\leqslant -0.5$ | 4 | 1 | 0 | 0 | 0 | 0 | 0 |
| | $\leqslant -0.4$ | 20 | 2 | 0 | 0 | 0 | 0 | 0 |
| | $\leqslant -0.3$ | 6 | 20 | 0 | 0 | 6 | 0 | 0 |
| | $\leqslant -0.2$ | 0 | 7 | 0 | 0 | 24 | 0 | 0 |
| | $\pm 0.1$ | 0 | 0 | 30 | 30 | 0 | 30 | 30 |
| | $\geqslant 0.2$ | 0 | 0 | 0 | 0 | 0 | 0 | 0 |
| one week | $\leqslant -0.5$ | 4 | 1 | 0 | 0 | 0 | 0 | 0 |
| | $\leqslant -0.4$ | 21 | 3 | 0 | 0 | 0 | 0 | 0 |
| | $\leqslant -0.3$ | 5 | 20 | 0 | 0 | 8 | 0 | 0 |
| | $\leqslant -0.2$ | 0 | 6 | 0 | 0 | 22 | 0 | 0 |
| | $\pm 0.1$ | 0 | 0 | 30 | 30 | 0 | 30 | 30 |
| | $\geqslant 0.2$ | 0 | 0 | 0 | 0 | 0 | 0 | 0 |
| two weeks | $\leqslant -0.5$ | 4 | 1 | 0 | 0 | 0 | 0 | 0 |
| | $\leqslant -0.4$ | 22 | 2 | 0 | 0 | 0 | 0 | 0 |
| | $\leqslant -0.3$ | 4 | 20 | 0 | 0 | 9 | 0 | 0 |
| | $\leqslant -0.2$ | 0 | 7 | 0 | 0 | 21 | 0 | 0 |
| | $\pm 0.1$ | 0 | 0 | 30 | 30 | 0 | 30 | 30 |
| | $\geqslant 0.2$ | 0 | 0 | 0 | 0 | 0 | 0 | 0 |

It can be seen from the above data that, at 72 hours after having taken-in the product, 4 people have a $\triangle F \leqslant$-0.5; 20 people have a $\triangle F$ -0.4; 6 people have a $\triangle F \leqslant$-0.3, among the 30 women having pathological change parts in breast,. It has therefore been proven that the product has obvious positive adjustment effect for female lobular hyperplasia, and has a good adjustment effect for endocrine disorder of adult lady, and no side effect on kidney and liver is observed.

Example 2

According to another embodiment of this invention, the same TTM apparatus as mentioned in Example 1 is used to evaluate the adjustment effect of a pill named as " Vitamin C Yin Qiao Pian", which is available from drugstore, for prevention of lung virus infection.

Taking 35 patients at an age of from 19 to 52 years old and having lung virus infection, which is characterized by having a temperature of 37.8-39.3°Cand is confirmed by clinical diagnosis, among them 20 are male, 15 are female. The pills are administrated by having it under the tongue, at a dosage of three times per day, three pills per time. The resulted data from TTM analysis are listed as follows:

| Time Interval | Check item \ Effect | $\Delta F_{lung}$ (Person) | $\Delta F_{axilla}$ (Person) | $\Delta F_{clavicle\ pit}$ (Person) | $\Delta F_{kidney}$ (Person) | $\Delta F_{gland}$ (Person) | $\Delta F_{liver}$ (Person) | $\Delta F_{stomach}$ or esophagues (Person)﹐ |
|---|---|---|---|---|---|---|---|---|
| 60 minutes | ≤-0.5 | 1 | 0 | 0 | 0 | 0 | 0 | 0 |
|  | ≤-0.4 | 6 | 2 | 1 | 0 | 0 | 0 | 0 |
|  | ≤-0.3 | 18 | 14 | 13 | 0 | 0 | 0 | 0 |
|  | ≤-0.2 | 10 | 19 | 21 | 0 | 6 | 0 | 0 |
|  | ±0.1 | 0 | 0 | 0 | 16 | 29 | 18 | 23 |
|  | ≥0.2 | 0 | 0 | 0 | 19 | 0 | 17 | 12 |
| 120 minutes | ≤-0.5 | 3 | 1 | 1 | 0 | 0 | 0 | 0 |
|  | ≤-0.4 | 12 | 5 | 6 | 0 | 0 | 0 | 0 |
|  | ≤-0.3 | 16 | 18 | 15 | 0 | 0 | 0 | 0 |
|  | ≤-0.2 | 4 | 11 | 13 | 0 | 8 | 0 | 0 |
|  | ±0.1 | 0 | 0 | 0 | 23 | 27 | 12 | 19 |
|  | ≥0.2 | 0 | 0 | 0 | 12 | 0 | 23 | 16 |
| 180 minutes | ≤-0.5 | 0 | 0 | 0 | 0 | 0 | 0 | 0 |
|  | ≤-0.4 | 2 | 2 | 0 | 0 | 0 | 0 | 0 |
|  | ≤-0.3 | 13 | 10 | 4 | 0 | 0 | 0 | 0 |
|  | ≤-0.2 | 18 | 20 | 26 | 0 | 2 | 0 | 0 |
|  | ±0.1 | 2 | 3 | 5 | 32 | 33 | 16 | 17 |
|  | ≥0.2 | 0 | 0 | 0 | 3 | 0 | 19 | 18 |

It can be seen from the data in above table that, within one to three hours after having orally taken-in three pills of Vitamin C Yin Qiao Pian", a best prohibitive effect for lung virus infection will appear, and the effect will gradually disappear after three hours. It can also be seen that this medicine has slight side effect on kidney, liver and alimentary canal, so it should not be taken for long term.

Example 3

According to another embodiment of this invention, the same TTM apparatus as mentioned in Example 1 is used to evaluate the adjustment effect of a physical treatment, i.e., a laser irradiation device model JX2000A, which is manufactured and available from Gueilin Kang Xin Medical Equipments Company Limited and is located at 2nd Floor, Gaoxin Chuangye Building, High-Tech Development Zone, Gueilin City, Guangxi Province, for improvement of the micro-circulatory state of human body. The laser irradiation device works at a laser wave-length of 650nm and has a laser output of 5mw. This treatment is made by a continuous irradiation in the nasal cavity lasting for 30 minutes. Among the 32 people having been subject to the irradiation, 12 are men and 20 are women. The resulted data from the TTM analysis are listed as follows:

$$( \triangle F_{finger\ tip} = F_{middle\ finger} - F_{finger\ tip} )\text{, and}$$

$$( \triangle F_{palm\ centre} = F_{left\ palm\ centre} - F_{right\ palm\ centre} )$$

| Time Interval | Check item / effect | $\triangle F_{finger\ tip}$ (Person) | $\triangle F_{palm\ centre}$ (Person) | $\triangle F_{clavicle\ pit}$ (Person) | $\triangle F_{axilla}$ (Person) | $\triangle F_{liver}$ (Person) | $\triangle F_{kidney}$ (Person) |
|---|---|---|---|---|---|---|---|
| 30 minutes | ≤-0.5 | 0 | 0 | 0 | 0 | 0 | 0 |
| | ≤-0.4 | 1 | 0 | 0 | 0 | 0 | 0 |
| | ≤-0.3 | 19 | 10 | 0 | 0 | 0 | 0 |
| | ≤-0.2 | 12 | 21 | 6 | 8 | 4 | 3 |
| | ±0.1 | 0 | 1 | 26 | 24 | 28 | 29 |
| | ≥0.2 | 0 | 0 | 0 | 0 | 0 | 0 |
| 60 minutes | ≤-0.5 | 0 | 0 | 0 | 0 | 0 | 0 |
| | ≤-0.4 | 0 | 0 | 0 | 0 | 0 | 0 |
| | ≤-0.3 | 12 | 3 | 0 | 0 | 0 | 0 |
| | ≤-0.2 | 19 | 23 | 3 | 4 | 3 | 2 |
| | ±0.1 | 1 | 6 | 29 | 28 | 29 | 30 |
| | ≥0.2 | 0 | 0 | 0 | 0 | 0 | 0 |
| 180 minutes | ≤-0.5 | 0 | 0 | 0 | 0 | 0 | 0 |
| | ≤-0.4 | 0 | 0 | 0 | 0 | 0 | 0 |
| | ≤-0.3 | 0 | 0 | 0 | 0 | 0 | 0 |
| | ≤-0.2 | 3 | 2 | 1 | 2 | 1 | 2 |
| | ±0.1 | 29 | 30 | 31 | 30 | 31 | 30 |
| | ≥0.2 | 0 | 0 | 0 | 0 | 0 | 0 |

It can be seen from the data listed in the above table that, at a time point of 30 minutes after a laser irradiation in the nasal cavity, a good improvement effect onto the micro-circulatory state of human body is observed, and the improvement effect could last for one to two hours after the irradiation is finished; meanwhile, it indicates no side effect onto liver and kidney.

**Claims**

1. , Evaluative method of effect of adjustment measures on human body's physiological and psychological states by means of using Thermal Texture Maps Technology (TTM), comprising the following steps:

   (1) Before certain adjustment measures are applied, conducting a scanning on the human body surface for a record of a thermal radiation mapping, taking a maximum value of the thermal radiation of each part, organs and tissues as its representative $F_i^0$, i = 1, 2, ... , I, in an unit of °C;I is the total number of the parts, organs and tissues, from which corresponding thermal radiation numerical values are measured by means of using TTM technology; thereby obtaining the thermal radiation intensity of the respective parts organs and tissues

$F_i^0$ , i = 1 , 2 , ... , I;

(2) Taking the thermal radiation numerical values of the lips, navel, and each endocrine glands, which are marked respectively as $F_{lips}^0$ , $F_{navel}^0$ , $F_{gland\,1}^0$ , 1= 1, ...L , L is the number of the endocrine glands to be tested; these $F_{lips}^0$ , $F_{navel}^0$ , $F_{gland\,1}^0$ , 1= 1, ...L are used to evaluate human body's original physiological states of human body as a whole before application of any adjustment measure; a healthy human body should has

$F_{lips}^0 = 0 \pm 0.2$ 、 , $F_{navel}^0 = 2.0 \pm 0.2$ 、 $F_{gland\,1}^0 \leq 0.1, 1 = 1, ...L;$

(3) Obtaining the nine thermal radiation values, marking them as $F_r^0$ , r =1, 2 , ... , 9, from the nine regional indicative windows, that is, two clavicle pit, two axillae and two inguina ditches on the left and right side of the body's centre axis respectively, and from the upper, middle and lower sector of the back spinal; Each parameter respectively stands for the original thermal radiation intensity of certain zone as follows:

$F_1^0$ :  left clavicle pit; $F_2^0$ :  right clavicle pit;

$F_3^0$ :  left axilla; $F_4^0$ :  right axilla;

$F_5^0$ :  left inguina ditch; $F_6^0$ :  right nguina ditch;

$F_7^0$ :  upper sector of the spinal $F_8^0$ :  middle sector of the spinal

$F_9^0$ :  lower sector of the spinal;

and figuring out the following 5 absolute differential values of the five symmetric pairs from the corresponding nine thermal radiation numerical values:

$$\Delta F_1^0 = \left| F_1^0 - F_2^0 \right|$$

$$\Delta F_2^0 = \left| F_3^0 - F_4^0 \right|$$

$$\Delta F_3^0 = \left| F_5^0 - F_6^0 \right|$$

$$\Delta F_4^0 = \left| F_7^0 - F_8^0 \right|$$

$$\Delta F_5^0 = \left| F_8^0 - F_9^0 \right|$$

if any one among $\Delta F_k^0$, k = 1 , ... , 5 is larger than or equal to 0.2, it then needs to further analyze and obtain, with help of TTM technology, the thermal radiation intensity of each and all parts, organs and tissues represented by the zone(s) having higher thermal radiation numerical value(s), so as to find out the parts organs and tissues having abnormal function, and to figure out the thermal radiation numerical values $F_{ot}^0$, **t** = 1, 2, ... , T ; T is the number of the abnormal parts, organs and tissues;

(4) Letting a human body accept one or more adjustment measures;

(5) In J time intervals, conducting scanning on human body surface for J records of the thermal radiation mapping with help of TTM technology, thereby obtaining J batches of the thermal radiation intensity of the respective parts

$$F_{唇}^j 、\ F_{脐}^j 、\ F_{腺1}^j \ , \ l = 1 \ , \ \dots \ , \ L \ ;$$

$$\Delta F_1^j 、\ \Delta F_2^j 、\ \Delta F_3^j 、\ \Delta F_4^j 、\ \Delta F_5^j \ ;$$

And

$$F_i^j \ , \ i=1 \ , \ 2 \ , \ \dots \ , \ I \ ;$$

$$j = 1 \ , \ 2 \ , \ \dots \ , \ J;$$

(6) Figuring out the followings:

$$\Delta F_{lips}^j = F_{lips}^j - F_{lips}^0$$

$$\Delta F_{navel}^j = F_{navel}^j - F_{腹navel}^0$$

$$\Delta F_{gland\,l}^j = F_{gland\,l}^j - F_{gland\,l}^0 \ , \ l = 1 \ , \ 2 \ , \ \dots \ , \ L;$$

$$\Delta(\Delta F_k)^j = \Delta F_k^j - \Delta F_k^0 \ , \ k = 1 \ , \ 2 \ , \ 3 \ , \ 4 \ , \ 5 \ ;$$

And

$$\Delta F_{ot}^{j} = F_{ot}^{j} - F_{ot}^{0} \; , \; t = 1 \; , \; 2 \; , \; \dots \; , \; T \; ;$$

$$j = 1 \; , \; 2 \; , \; \dots \; , \; J \; ;$$

(7) As for the newly found Q parts having abnormal function clued by obviously increased $\Delta(\Delta F_k)^j$, k = 1 , 2 , 3 , 4 , 5 ; j = 1 , 2 , ... J in step (6), conducting TTM analysis and determining the followings:

$$\Delta F_{q}^{j} = F_{q}^{j} - F_{q}^{0} \; , \; q = 1 \; , \; 2 \; , \; \dots \; , \; Q \; ; \; j = 1 \; , \; 2 \; , \; \dots \; , \; J \; ;$$

(8) Using the differential values of thermal radiation intensity $\Delta F^j$ and $\Delta(\Delta F)^j$, j = 1 , ...J, obtained in steps (6) and (7), for the purpose of determining the positive and side effect of the adjustment measures applied onto the human body as a whole, onto regional zones and individual organs, tissues, and determining the starting time point and the lasting period of the effectuation, according to the criteria below:

$0\leq$ and $\leq 0.1$, having side effect, but not obvious
$>0.1$ and $\leq 0.2$, obvious side effect
$>0.2$ and $\leq 0.3$, strong side effect
$>0.3$ and $\leq 0.4$, very strong side effect
$\geq 0.5$ poison;
$\leq 0$ and $\geq -0.1$, positive effect, not clear
$<-0.1$ and $\geq -0.2$, obvious positive effect
$<-0.2$ and $\geq -0.3$, strong positive effect
$<-0.3$ and $\geq -0.4$, very strong positive effect
$< -0.5$ best effect.

**2.** The method according to claim 1, **characterized in that**, the said "adjustment measures" include any of taken-in substances, physical stimulations and mental treatments or their ambiguous combinations which are actively applied to or are passively accepted by human body and which could bring about changes to physiological and psychological states of human body.

**3.** The method according to claim 2, **characterized in that**, the said adjustment measures are any one or more of those substances which are taken-in actively or passively by human body and are selected from the group comprising medicine, tonics, nutriment, body care products such as cosmetics, beauty making-up, etc., enhancer agent, food therapy articles; cigarette smoking, alcohol, harmful substances which cause dependence of human body to them or cause obviously disturbs to human body's normal physiological and psychological functions; pollutants, toxic substances contained in atmosphere, drinking water, and/or food; misuse, overuse of the medicine, tonics, nutriment, body care products, enhancer agent, food therapy articles; the said taking-in comprises oral administration, injection, percutaneous adsorption, smoking and respiration.

**4.** The method according to claim 2, **characterized in that**, the said adjustment measures are any one or more of those physical stimulations which are actively applied to or passively accepted by human body and are selected from the group comprising keep-fit massage, health-aimed sports, acupuncture-moxibustion, digit-pressing moxibustion, electromagnetic spectrum therapy, needle embedment, meditation and qigong exercise; electromagnetic field-, light- and radioactive pollution; (ultra)sound wave and noise pollution; harms and disability caused by outside force; harmful living and working conditions, habits and ways.

**5.** The method according to claim 2, **characterized in that**, the said adjustment measures are any one or more of those mental treatments which are actively applied to or passively accepted by human body and are selected from the group comprising moral encouragement, consolation, physiological therapy, hypnotism, religious reformatory; fright, threatening and irrational mental control, as well as the unhealthy environment or relationship with other people which could lead to a mood of constrain, sorrow, decline, despair, hatred; IQ test and lie detection.

6. The method according to claim 3, **characterized in that**, the said substances are selected from the group comprising medicine, tonics, nutriment, body care products and food therapy articles.

7. The method according to claim 6, **characterized in that**, the said substance is "Rou Zhi Mei emulsifiable particulate" made from natural plants for preventing hyperplasia of mammary gland.

8. The method according to claim 6, **characterized in that**, the said substance is a pills named as " Vitamin C Yin Qiao Pian".

9. The method according to claim 4, **characterized in that**, the said physical stimulation is a laser irradiation for improvement of the micro-circulatory state of human body.

10. Use of TTM technology in evaluation of effect of adjustment measures on the human body's physiological and psychological states.

## INTERNATIONAL SEARCH REPORT

| | International application No. |
|---|---|
| | PCT/CN2005/000290 |

**A. CLASSIFICATION OF SUBJECT MATTER**

IPC⁷ A61B 5/00

According to International Patent Classification (IPC) or to both national classification and IPC

**B. FIELDS SEARCHED**

Minimum documentation searched (classification system followed by classification symbols)

IPC⁷ A61B 5/+

Documentation searched other than minimum documentation to the extent that such documents are included in the fields searched

CNPAT(1985-2005)

Electronic data base consulted during the international search (name of data base and, where practicable, search terms used)

WPI,EPODOC,PAJ

**C. DOCUMENTS CONSIDERED TO BE RELEVANT**

| Category* | Citation of document, with indication, where appropriate, of the relevant passages | Relevant to claim No. |
|---|---|---|
| A | CN,Y,2542208(LIU Zhongqi et al)2.Apr 2003(04.02.2003) the whole document | 1,10 |
| A | CN,Y,2549905(LIU Zhongqi et al)14.May 2003(14.05.2003) the whole document | 1,10 |
| A | WO,A,9846976(LIU Zhongqi et al)22.Oct 1998(22.10.1998) the whole document | 1,10 |
| A | WO,A,0101854(HYPERMED IMAGING,INC)11.Jan 2001(11.01.2001) the whole document | 1,10 |

☐ Further documents are listed in the continuation of Box C.     ☒ See patent family annex.

| *   Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
|---|---|
| 27.May 2005(27.05.2005) | 23 · JUN 2005 (2 3 · 0 6 · 2 0 0 5) |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>           Qiwei Zheng<br>Telephone No. 86-10-62085797 |

Form PCT/ISA /210 (second sheet) (April 2005)

# EP 1 723 904 A1

| INTERNATIONAL SEARCH REPORT | International application No. |
|---|---|
| | PCT/CN2005/000290 |

**Box No. II    Observations where certain claims were found unsearchable (Continuation of item 2 of first sheet)**

This international search report has not been established in respect of certain claims under Article 17(2)(a) for the following reasons:

1. ☒   Claims Nos.:2-9
because they relate to subject matter not required to be searched by this Authority, namely:
claims 2-9 concern methods for the treatment of diseases, so they fall under Rule 67.1(iv)PCT.

2. ☐   Claims Nos.:
because they relate to parts of the international application that do not comply with the prescribed requirements to such an extent that no meaningful international search can be carried out, specifically:

3. ☐   Claims Nos.:
because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

1. ☐   As all required additional search fees were timely paid by the applicant, this international search report covers all searchable claims.

2. ☐   As all searchable claims could be searched without effort justifying an additional fees, this Authority did not invite payment of any additional fee.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on protest**        ☐   The additional search fees were accompanied by the applicant's protest and, where applicable, the payment of a protest fee.

☐   The additional search fees were accompanied by the applicant's protest but the applicable protest fee was not paid within the time limit specified in the invitation.

☐   No protest accompanied the payment of additional search fees.

Form PCT/ISA/210 (continuation of first sheet (2)) (April 2005)

| INTERNATIONAL SEARCH REPORT | International application No. |
| :---: | :---: |
| Information on patent family members | PCT/CN2005/000290 |

| Patent document cited in search report | Publication date | Patent family Members | Publication Date |
| :---: | :---: | :---: | :---: |
| CN,Y,2542208 | 02.04.2003 | None | |
| CN,Y,2549905 | 14.05.2003 | None | |
| WO,A,9846976 | 22.10.1998 | AU,A,8642898 | 11.11.1998 |
| | | US,A,6023637 | 08.02.2000 |
| WO,A,0101854 | 11.01.2001 | AU,A,200057839 | 22.01.2001 |
| | | EP,A,1196081 | 17.04.2002 |
| | | JP,T,2003503135 | 28.01.2003 |
| | | US,B,6640130 | 28.10.2003 |

Form PCT/ISA/210(patent family annex)(July 1992)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6023637 A
- CN ZL02235271
- CN ZL02234963
- CN ZL02308058
- CN 03276575
- CN 03276576